# FASCICULE DE BREVET EUROPEEN

(11) **EP 1 198 205 B1**
(45) Date de publication et mention de la délivrance du brevet: **15.11.2006**
(21) Numéro de dépôt: 00956560.7
(22) Date de dépôt: 20.07.2000
(51) Int. Cl.: A61B 17/70

(54) **LIAISON POLY-AXIALE POUR OSTEOSYNTHESE**
OSTEOSYNTHETISCHE POLYAXIALVERBINDUNG
MULTIAXIAL CONNECTION FOR OSTEOSYNTHESIS

(30) Priorité: 22.07.1999 FR 9909524
(43) Date de publication de la demande: 24.04.2002
(73) Titulaire: Stryker Spine, 33610 Cestas (FR)
(72) Inventeur: BACCELLI, Christian, F-33640 Ayguemorte les Graves (FR); CONCHY, Frédéric, F-33650 Saint Médard d'Eyrans (FR); GAUCHET, Fabien, F-60800 Duvy (FR); LE COUEDIC, Régis, F-33610 Cestas (FR); PASQUET, Denis, F-33000 Bordeaux (FR); SAINT MARTIN, Pierre, H., Rés. "les Jardins, F-33600 Pessac (FR); TURNER-DOMERGUE, Michèle, F-33600 Pessac (FR); VIENNEY, Cécile, F-33000 Bordeaux (FR)
(74) Mandataire: Le Forestier, Eric
(86) Numéro de dépôt international: PCT/FR2000/002085
(87) Numéro de publication internationale: WO 2001/006940

(56) Documents cités:
- WO-A-97/02786
- WO-A-98/32386
- DE-U- 29 903 342
- FR-A- 2 771 918

## Description

La présente invention concerne un système d'ostéosynthèse à liaison poly-axiale, notamment pour le rachis.

Dans le cadre de la chirurgie de la colonne vertébrale, par exemple, et notamment par voie d'approche postérieure, le praticien est souvent confronté au problème du mésalignement des organes d'ancrages ainsi que de l'interférence entre de tels organes sur deux vertèbres adjacentes. Ce problème a pour conséquence d'allonger le temps opératoire ou de ne pas permettre au praticien, dans les meilleures conditions qui soient, de réaliser l'intervention désirée. La source de ce problème est que l'élément de couplage entre l'organe de liaison des différentes vertèbres et l'organe d'ancrage dans la vertèbre est rigidement lié à cet organe d'ancrage.

Le document N° WO 97/02786 nous enseigne une liaison poly-axiale entre les éléments. L'organe d'ancrage comporte une tête semi-sphérique. L'élément de couplage comporte, en plus des moyens pour recevoir l'organe de liaison des différentes vertèbres, une chambre fendue de réception de la tête semi-sphérique dans sa partie inférieure. La surface externe de l'élément de couplage comporte un cône s'évasant vers l'extrémité inférieure. Un collier de serrage est apte à s'enfiler sur l'élément de couplage par sa partie supérieure pour venir s'appuyer sur la partie conique. Avant serrage, l'élément de couplage est libre de rotation par rapport à l'organe d'ancrage. Le blocage en position s'effectue lors du serrage de l'organe de liaison des différentes vertèbres. Ce dernier vient appuyer sur le collier de serrage qui referme dans le même temps la chambre de réception sur la tête semi-sphérique de l'organe d'ancrage.

La complexité du mécanisme de blocage en position, du fait de ses nombreuses pièces, oblige le praticien à effectuer des manipulations supplémentaires, ce qui a pour conséquence d'allonger les temps opératoires.

Un but de l'invention est de fournir un dispositif de liaison poly-axiale simple de mise en oeuvre, pouvant être mis en place avec un nombre réduit d'opérations.

En vue de réaliser ce but, on prévoit, selon la présente invention, un système d'ostéosynthèse selon la revendication 1.

Ainsi, le premier logement déformable se referme sur la tête. Cela permet de réduire le nombre de pièces, donc de simplifier la mise en oeuvre du dispositif.

Avantageusement, le premier logement comporte une chambre à surface concave.

Avantageusement, la chambre à surface concave est complémentaire de tout ou partie de la surface convexe de la tête.

Avantageusement, le premier logement comporte au moins une cavité apte à recevoir en partie la tête.

Avantageusement, le connecteur comporte au moins une fente s'étendant dans le premier logement.

Ainsi, le premier logement pourra avoir une déformabilité élastique plus prononcée lors de l'introduction de la tête et lors du blocage en position du dispositif.

Avantageusement, la tête comporte une partie sphérique.

Avantageusement, le premier logement comporte une ouverture en « U » comprenant une axe et deux branches s'étendant à distance et en regard l'une de l'autre.

Avantageusement, la fente est perpendiculaires à l'axe de l'ouverture en " U ".

Avantageusement les branches de l'ouverture en " U " présentent un filetage.

Avantageusement, les moyens de serrage comportent un verrou apte à venir en prise entre les branches de l'ouverture en " U ".

Avantageusement, les moyens de serrage comportent une bride apte à venir autour des branches de l'ouverture en " U ".

Ainsi, lors du serrage du verrou, la bride empêche l'écartement des branches de l'ouverture en " U ".

Avantageusement, le filetage comporte une face sensiblement perpendiculaire à l'axe du filet et orientée suivant un sens de pénétration du moyen de serrage sur le connecteur.

Ainsi, lors du serrage, la face sensiblement perpendiculaire à l'axe du filet du verrou entrent en contact avec celle des branches de l'ouverture en « U ». La force de réaction ainsi provoquée est sensiblement parallèle à l'axe du filetage et on minimise la force de réaction radiale qui ferait, sinon, s'écarter les branches de l'ouverture en " U " .

D'autres caractéristiques et avantages de l'invention apparaîtront encore dans la description suivante de trois modes de réalisation préférés donnés à titre d'exemples non limitatifs. Aux dessins annexés :
- la figure 1 est une vue partielle en perspective du système selon une première réalisation ;
- la figure 2 est une vue en perspective éclatée du système de la figure 1 ;
- la figure 3 est une vue en coupe du système selon le plan III-III de la figure 1 ;
- la figure 4 est une vue en perspective de dessus du connecteur selon la première réalisation ;
- la figure 5 est une vue en perspective de dessous du connecteur de la figure 4 ;
- la figure 6 est une vue en coupe du connecteur selon le plan VI-VI de la figure 4 ;
- la figure 6a est une vue schématique en coupe du filetage en sapin de Noël du connecteur de la figure 4 ;
- la figure 7 est une vue en perspective de dessous du connecteur selon une seconde réalisation ; et
- la figure 8 est une vue en perspective de dessous du connecteur selon une troisième réalisation.

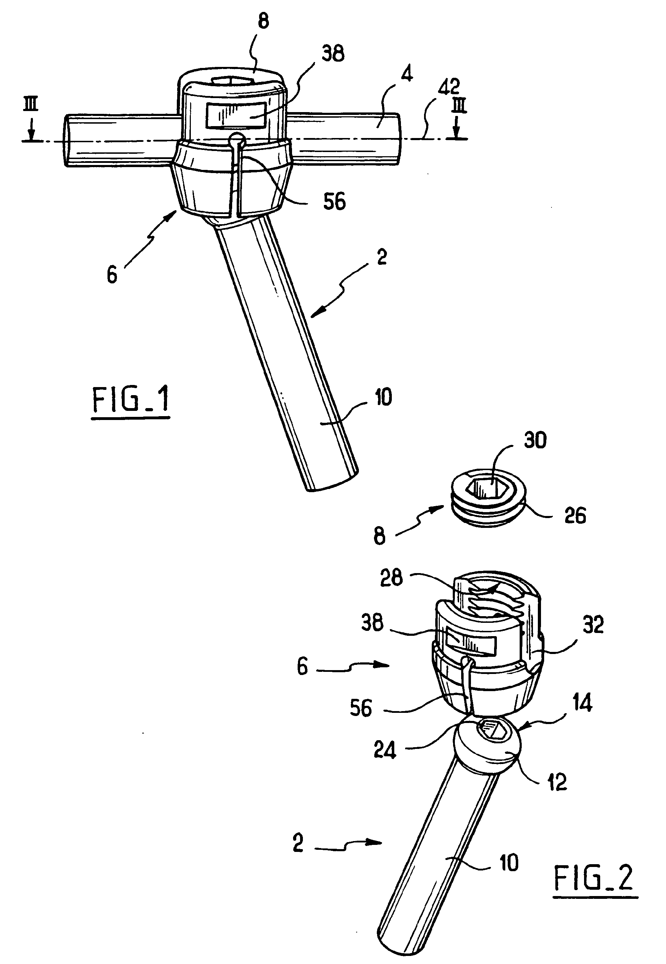

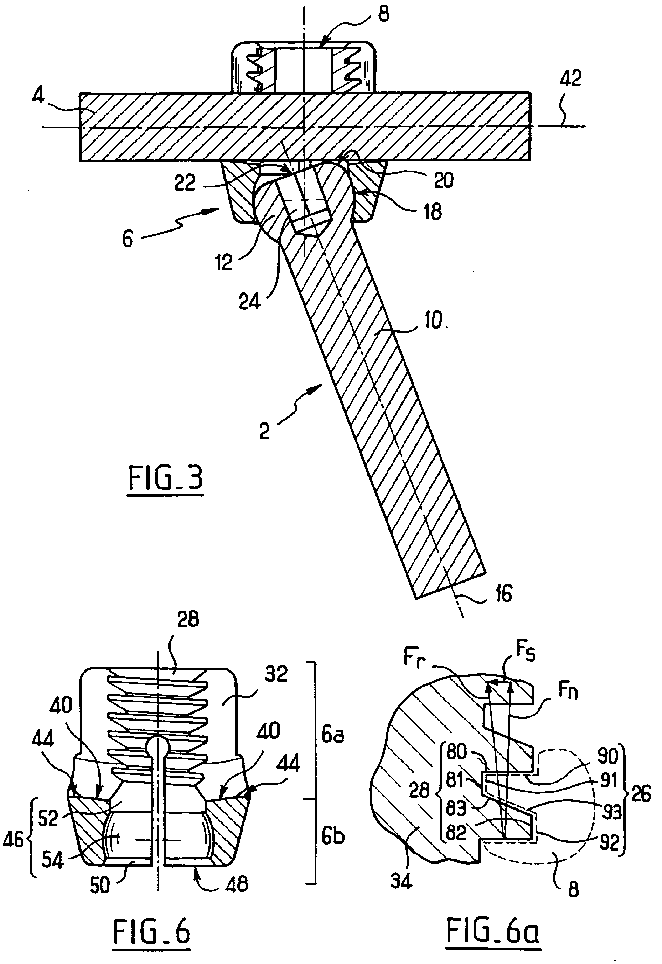

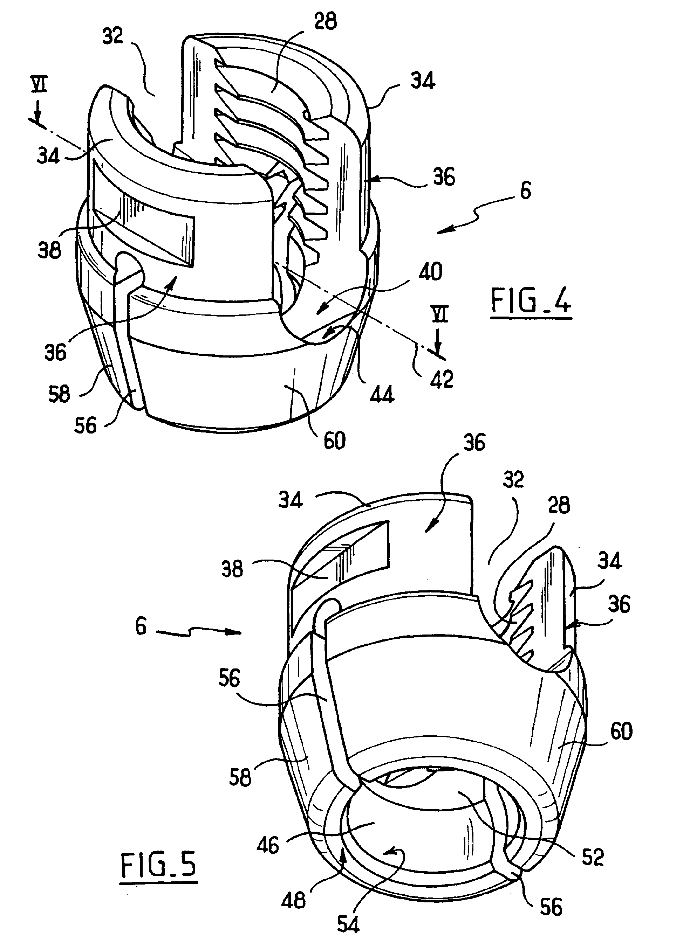

Nous allons décrire un premier mode de réalisation de la présente invention en référence aux figures 1 à 6a.

Le système d'ostéosynthèse comprend au moins deux organes d'ancrage 2 vertébral, des moyens de liaison 4, tels qu'une tige, entre cet organe d'ancrage 2 et les autres organes d'ancrages (non représentés) du système d'ostéosynthèse, un connecteur 6 apte à venir s'adapter sur l'organe 2, et un verrou 8 apte à coopérer avec le connecteur 6.

L'organe d'ancrage vertébral 2, ici sous la forme d'une vis à os, comprend un corps fileté 10 cylindrique de section circulaire ayant un filetage à os (non représenté). L'organe comporte aussi une tête 12 présentant une surface convexe 14, ici une surface de révolution autour de l'axe de la vis 16. La surface convexe comporte une partie latérale sphérique 18, une partie supérieure plane 22 perpendiculaire à l'axe de la vis, et un congé de raccordement 20 entre celles-ci. La tête 12 comporte des moyens de mise en oeuvre 24 de la vis 2, ici sous la forme d'une empreinte 24 en creux ayant six pans et de forme hexagonale.

Le verrou a ici une forme générale de vis et comporte un filetage 26 apte à coopérer avec celui 28 présent sur le connecteur 6 décrit ci-après. Le verrou comporte aussi des moyens de mise en oeuvre 30 ici sous la forme d'une empreinte 30 à six pans de forme hexagonale.

Le connecteur 6 est un élément de couplage entre la vis à os 2 et la tige 4 de liaison. Cet élément de couplage est de forme générale cylindrique à section circulaire. Il comporte une partie supérieure 6a pour la liaison avec la tige 4 et une partie inférieure 6b pour la liaison avec la vis à os 2.

Dans la partie supérieure 6a, l'élément de couplage 6 comprend une ouverture 32 en " U " ayant un axe 42 et délimitée par deux branches 34 s'étendant à distance et en regard l'une de l'autre. Ces deux branches 34 constituent deux secteurs d'un même cylindre à section circulaire formant l'élément de couplage 6. Les faces externes cylindriques 36 des branches 34 sont coaxiales et lisses. Elles peuvent comporter des moyens de préhension 38 par exemple sous forme d'une encoche à fond plat parallèle à l'axe 42 de l'ouverture en "U " 32 et situé sur chacune des branches 34. Les encoches 38 sont symétriques en miroir par rapport au plan médian de l'ouverture en « U » 32. Les faces internes cylindriques 28 des branches sont coaxiales et filetées. Ce filetage est apte à coopérer avec celui du verrou 26. Les deux filetages 26 et 28 sont complémentaires et sont dits " en sapin de Noël ". Le filetage 28 présente une première face 80 sensiblement perpendiculaire à l'axe du filet, une seconde face 81 sensiblement parallèle à l'axe du filet délimitant le fond du filet, une troisième face 83 s'étendant en regard de la première face 80 et pouvant être inclinée en direction de l'axe par rapport à la direction de la première face 80, et une quatrième face 82 sensiblement parallèle à l'axe du filet délimitant la crête du filet. Le filetage 26 est sensiblement complémentaire du filetage 28. Ce filetage 26 présente une première face 90 parallèle et complémentaire de la face 80, une seconde face 91 sensiblement parallèle à l'axe du filet délimitant la crête du filet, une troisième face 93 sensiblement parallèle et complémentaire de la face 83, et une quatrième face 92 sensiblement parallèle à l'axe du filet délimitant le fond du filet. Lors du serrage, la face 90 du filetage 28 vient en contact avec la face 80 du filetage 26. La force de réaction Fr ainsi obtenue se décompose en une force normale Fn, parallèle à l'axe des file, et en une force transversale Fs de direction radiale. Cette force transversale Fs pourrait être responsable de l'écartement des branches 34 lors du serrage du verrou 8, mais la forme particulière, précitée, des filetages 26,28 permet de réduire la valeur de la force transversale Fs, et ainsi de réduire l'écartement des branches 34 au moment du serrage.

Dans un plan perpendiculaire à l'axe 42, le fond 40 de l'ouverture en " U " est semi-circulaire pour pouvoir recevoir la tige de liaison 4, avec un diamètre équivalent à celui de la tige de liaison 4. D'autre part, le fond 40 présente une concavité dans le plan médian de l'ouverture 32 en « U » et ayant un centre de courbure situé du coté de la partie 6a du connecteur 6. Enfin, le fond 40 présente des extrémités 44 parallèles à l'axe 42, servant de siège de réception pour la tige de liaison 4.

Dans la partie inférieure 6b, le connecteur 6 comprend un logement 46 débouchant sur la face inférieure 48 en un orifice 50 d'insertion et débouchant dans le fond 40 de l'ouverture en « U » 32 en un orifice de communication 52. L'orifice d'insertion 50 de section circulaire permet l'introduction de la tête 12 de la vis à os 2 dans le logement 46. Le logement 46 comporte une chambre inférieure 54 en portion de sphère qui est apte à recevoir la tête 12 de la vis à os 2. La forme de la chambre inférieure 54 est complémentaire de la portion latérale sphérique 18 de la surface convexe 14 de la tête 12. Une fente 56 traverse diamétralement la partie inférieure 6b. La fente 56 est perpendiculaire à l'axe 42 de l'ouverture en « U » 32. Elle s'étend, vers le bas, jusqu'à la face inférieure 48 du connecteur 6 et, vers le haut, jusqu'à déboucher dans le fond 40 de l'ouverture en « U » 32. Ainsi la partie inférieure 6b est partagée en deux sous-parties 58 et 60 symétriques en miroir par rapport au plan médian de la fente 56. La fente 56 facilite le clipsage de la tête 12 dans l'élément de couplage 6 en facilitant la déformabilité de l'espace de réception 46.

Avant l'intervention, chaque connecteur 6 est prémonté par clipsage sur la tête 12 de la vis à os 2. La vis à os est mise en place sur le patient en utilisant les moyens de mise en oeuvre 24 qu'un instrument atteint via l'orifice de communication 52. Une fois la vis 2 mise en place, le connecteur 6 est libre de rotation par rapport à cette vis 2. Ceux-ci sont reliés l'un à l'autre par une liaison rotule formée par la tête 12 et la chambre inférieure 54. La tige 4 est mise en place dans l'ouverture en " U" 32 de manière à venir reposer sur les extrémités 44 du fond 40. Le verrou 8 est ensuite engagé entre les branches 34 de l'ouverture 32, les filetages 26 et 28 en prise l'un avec l'autre. Le verrou 8 est amené alors en appui contre la tige 4 et le praticien serre le verrou 8. Le verrou 8 appuie sur la tige 4. La tige 4 appuie sur les extrémités 44 du fond 40 qui se déforme alors grâce à la présence de la fente 56 perpendiculaire à l'axe 42 de l'ouverture 32 qui se referme en rapprochant les deux sous-parties 58 et 60 l'une de l'autre. Par conséquent, l'espace de réception 46 et la chambre 54 se referment sur la tête 12 de la vis à os 2, bloquant l'ensemble en position, immobilisant rigidement le connecteur sur la tête de la vis à os.

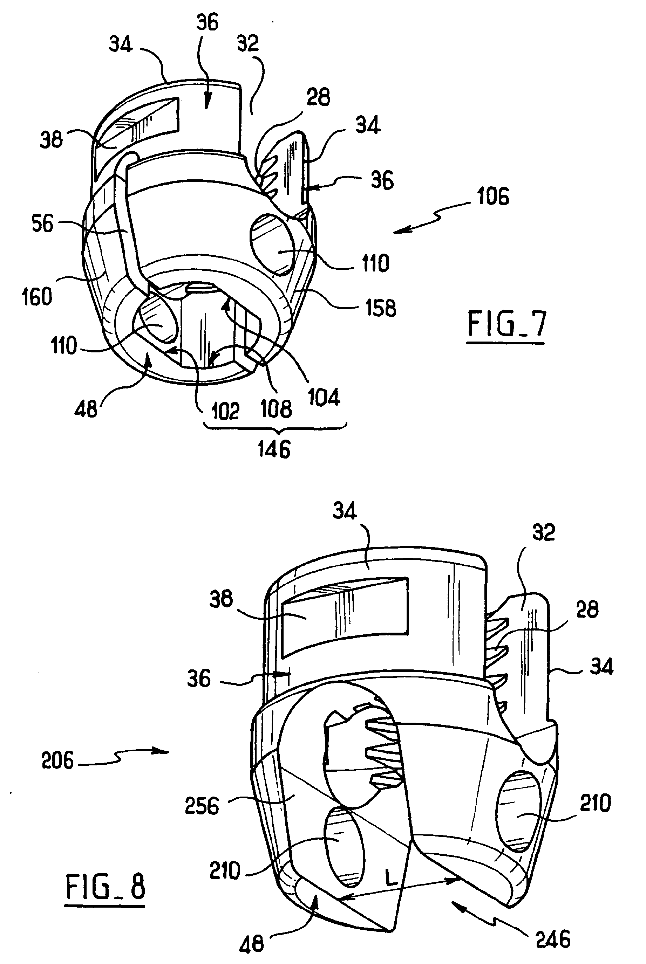

Dans le deuxième mode de réalisation illustré à la figure 7, les modifications par rapport au premier mode de réalisation concernent la partie inférieure du connecteur 106. Le logement 146 ne comporte plus de chambre sphérique mais est de forme cylindrique circulaire avec deux méplats 102 et 104. Le rayon de la partie circulaire 108 pourra être équivalent au rayon de la tête 12 de la vis à os 2. Les deux méplats 102 et 104 sont parallèles à la fente 56. Perpendiculairement à ces méplats 102 et 104, un trou 110 de section circulaire traverse diamétralement la partie inférieure de part en part au niveau des méplats, perpendiculairement à ceux-ci. Le diamètre de ce trou 110 est sensiblement équivalent à la largeur des méplats 102 et 104.

Lors du clipsage du connecteur 106 sur la tête 12 de la vis à os 2, la tête 12 vient se loger dans les trous 110 laissant libre la liaison rotule ainsi formée.

L'utilisation est identique à celle du premier mode de réalisation lors du serrage, la déformation de l'espace de réception 146, par le rapprochement des deux sous-parties 158 et 160 l'une vers l'autre, oblige les deux méplats 102 et 104 à se rapprocher l'un de l'autre, bloquant, de ce fait, la tête 12 de la vis à os 2 dans les trous 110.

Dans le troisième mode de réalisation illustré à la figure 8, les modifications par rapport au mode précédent concernent la partie inférieure du connecteur 206. Le logement 246 et la fente 256 sont confondus, la fente 256 ayant la largeur du logement 246. La largeur L de la fente 256 est inférieure au diamètre de la tête 12 de la vis à os 2. Comme dans le mode de réalisation précédent, un trou 210 de section circulaire traverse diamétralement, de manière perpendiculaire aux parois de la fente 256, la partie inférieure du connecteur 206 de part en part au niveau des méplats, perpendiculairement à ceux-ci. Le diamètre de ce trou 210 est inférieur à celui de la partie sphérique 18 de la surface convexe 14 de la tête 12.

Le clipsage s'effectue de la même manière : la tête 12 vient se loger dans les trous 210 laissant libre la liaison rotule ainsi formée.

L'utilisation est identique à celle du second mode de réalisation. Le blocage en position est similaire.

Bien entendu, on pourra apporter à l'invention de nombreuses modifications sans sortir du cadre de celle-ci.

Les organes d'ancrage pourraient être des crochets. Le moyen de serrage pourra comporter une bague ou une bride apte à s'enfiler sur les branches de l'ouverture en " U " en remplacement du verrou ou en complément de ce dernier. Dans ce cas de complément, cette bride ou bague pourra être liée au verrou.

Le filet coopérant avec celui des branches pourra être sur la bride, les branches étant filetées sur leur face extérieure.

## Revendications

1. Système d'ostéosynthèse à liaison poly-axiale notamment pour le rachis, comprenant un élément d'ancrage osseux (2) comportant une tête (12), un élément de liaison (4), et un connecteur (6;106;206) présentant un premier logement déformable (46;146;246) pour recevoir la tête et un deuxième logement (32) pour recevoir l'élément de liaison, le système comprenant un moyen de serrage (8) de l'élément de liaison dans le deuxième logement, et un siège (44) de réception pour l'élément de liaison **caractérisé en ce que** le siège est d'une seule pièce avec le connecteur et le connecteur est agencé de sorte que, lorsque le moyen de serrage sollicite l'élément de liaison dans le deuxième logement, l'élément de liaison sollicite directement le siège du connecteur pour déformer le premier logement et y bloquer la tête.

2. Système selon la revendication 1 **caractérisé en ce que** le premier logement comporte une chambre (54) à surface concave.

3. Système selon la revendication 1 ou 2 **caractérisé en ce que** le premier logement comporte au moins une cavité (110;210) apte à recevoir en partie la tête (12).

4. Système selon l'une des revendications 1 à 3 **caractérisé en ce que** la tête comprend une partie sphérique (18).

5. Système selon l'une des revendications 1 à 4 **caractérisé en ce que** le connecteur comporte au moins une fente (56;256) s'étendant dans le premier logement.

6. Système selon l'une des revendications 1 à 5 **caractérisé en ce que** le premier logement présente une ouverture en « U » comprenant un axe (42) et deux branches (34) s'étendant à distance et en regard l'une de l'autre.

7. Système selon les revendications 5 et 6 **caractérisé en ce que** la fente est perpendiculaires à l'axe (42) de l'ouverture en " U ".

8. Système selon la revendication 6 ou 7 **caractérisé en ce que** le moyen de serrage comportent un verrou (8) apte à venir en prise entre les branches de l'ouverture en " U".

9. Système selon l'une des revendications 6 à 8 **caractérisé en ce que** le moyen de serrage comporte une bride apte à venir autour des branches de l'ouverture en " U ".

10. Système selon l'une des revendications 6 à 9 **caractérisé en ce que** les branches (34) de l'ouverture en " U " présentent un filetage (28).

11. Système selon la revendication 10 **caractérisé en ce que** le filetage (26,28) comporte une face (80) sensiblement perpendiculaire à l'axe du filet et orientée suivant un sens de pénétration du moyen de serrage sur le connecteur.

## Claims

1. Multiaxial connection osteosynthesis system, in particular for the spine, including a bone anchor member (2) having a head (12), a connecting member (4), and a connector (6; 106; 206) having a first deformable housing (46; 146; 246) adapted to receive the head and a second housing (32) adapted to receive the connecting member, the system including clamping means (8) for clamping the connecting member in the second housing, and a seat (44) for receiving the connecting member, **characterized in that** the seat is made in one piece with the connector, and the connector is arranged so that, when the clamping means load the connecting member in the second housing, the connecting member loads the connector seat directly to deform the first housing and immobilize the head therein.

2. System according to Claim 1, **characterized in that** the first housing includes a chamber (54) with a concave surface.

3. System according to Claim 1 or 2, **characterized in that** the first housing includes a cavity (110; 210) adapted to receive part of the head (12).

4. System according to one of Claims 1 to 3, **characterized in that** the head has a spherical part (18).

5. System according to one of Claims 1 to 4, **characterized in that** the connector includes a slot (56; 256) in the first housing.

6. System according to one of Claims 1 to 5, **characterized in that** the first housing has a U-shaped opening having an axis (42) and two branches (34) at a distance from and facing each other.

7. System according to Claims 5 and 6, **characterized in that** the slot is perpendicular to the axis (42) of the U-shaped opening.

8. System according to Claim 6 or 7, **characterized in that** the clamping means include a locking member (8) adapted to engage between the branches of the U-shaped opening.

9. System according to one of Claims 6 to 8, **characterized in that** the clamping means includes a flange adapted to fit around the branches of the U-shaped opening.

10. System according to one of Claims 6 to 9, **characterized in that** the branches (34) of the U-shaped opening have a screwthread (28).

11. System according to Claim 10, **characterized in that** the screwthread (26, 28) has a face (80) substantially perpendicular to the axis of the thread and oriented in a penetration direction of the clamping means on the connector.

## Patentansprüche

1. Osteosynthesesystem mit mehrachsiger Verbindung, insbesondere für die Wirbelsäule, das folgendes umfaßt: ein Element zur Verankerung im Knochen (2), das einen Kopf (12) aufweist, ein Verbindungselement (4) und ein Kupplungsteil (6; 106; 206), das ein erstes verformbares Lager (46; 146; 246) zur Aufnahme des Kopfs und ein zweites Lager (32) zur Aufnahme des Verbindungselements umfaßt, wobei das System ein Mittel zum Fesklemmen (8) des Verbindungselements im zweiten Lager und einen Sitz (44) zur Aufnahme des Verbindungselements aufweist, **dadurch gekennzeichnet, daß** der Sitz einstückig mit dem Kupplungsteil ausgeführt ist und das Kupplungsteil derart eingerichtet ist, daß wenn das Feststellmittel auf das Verbindungselement im zweiten Lager einwirkt, das Verbindungselement direkt auf den Sitz des Kupplungsteils einwirkt, um das erste Lager zu verformen und dort den Kopf zu blockieren.

2. System nach Anspruch 1, **dadurch gekennzeichnet, daß** das erste Lager eine Kammer (54) mit konkaver Oberfläche umfaßt.

3. System nach Anspruch 1 oder 2, **dadurch gekennzeichnet, daß** das erste Lager wenigstens eine Aushöhlung (110; 210) umfaßt, die dafür eingerichtet ist, den Kopf (12) teilweise aufzunehmen.

4. System nach einem der Ansprüche 1 bis 3, **dadurch gekennzeichnet, daß** der Kopf einen kugelförmigen Abschnitt (18) umfaßt.

5. System nach einem der Ansprüche 1 bis 4, **dadurch gekennzeichnet, daß** das Kupplungsteil wenigstens einen Schlitz (56; 256) umfaßt, der sich im ersten Lager erstreckt.

6. System nach einem der Ansprüche 1 bis 5, **dadurch gekennzeichnet, daß** das erste Lager eine Öffnung in "U"-Form hat, die eine Achse (42) umfaßt sowie zwei Schenkel (34), die sich in einem Abstand und einander gegenüberstehend erstrecken.

7. System nach den Ansprüchen 5 und 6, **dadurch gekennzeichnet, daß** der Schlitz senkrecht zur Achse (42) der "U"-förmigen Öffnung ist.

8. System nach Anspruch 6 oder 7, **dadurch gekennzeichnet, daß** das Festklemmittel ein Blockierelement (8) umfaßt, das dafür eingerichtet ist, zwischen den Schenkeln der "U"-förmigen Öffnung in Eingriff zu kommen.

9. System nach einem der Ansprüche 6 bis 8, **dadurch gekennzeichnet, daß** das Festklemmittel ein Ringelement umfaßt, daß dafür eingerichtet ist, um die Schenkel der "U"-förmigen Öffnung herum zu sitzen.

10. System nach einem der Ansprüche 6 bis 9, **dadurch gekennzeichnet, daß** die Schenkel (34) der "U"-förmigen Öffnung ein Gewinde (28) haben.

11. System nach Anspruch 10, **dadurch gekennzeichnet, daß** das Gewinde (26, 28) eine Fläche (80) hat, die im wesentlichen senkrecht zur Achse des Gewindes ist und die in einer Eindringrichtung des Mittels zum Festklemmen am Kupplungsteil orientiert ist.
